# EUROPÄISCHE PATENTSCHRIFT

(11) **EP 0 816 831 B1**
(45) Veröffentlichungstag und Bekanntmachung des Hinweises auf die Patenterteilung: **14.05.2003**
(21) Anmeldenummer: 97112668.5
(22) Anmeldetag: 18.03.1994
(51) Int. Cl.: G01N 21/86

(54) **Teststreifenauswertegerät**
Device for evaluating test-strips
Dispositif pour l'évaluation de bandes de test

(30) Priorität: 31.03.1993 DE 4310583
(43) Veröffentlichungstag der Anmeldung: 07.01.1998
(62) Teilanmeldung aus: 94104267.3
(73) Patentinhaber: Roche Diagnostics GmbH, 68305 Mannheim (DE)
(72) Erfinder: Hönes, Joachim, 64673 Zwingenberg (DE); Unkrig, Volker, Dr., 68526 Ladenburg (DE); Steeg, Klaus-Dieter, 76709 Kronau (DE)
(74) Vertreter: Pfeifer, Hans-Peter, Dr.

(56) Entgegenhaltungen:
- EP-A- 0 129 220
- EP-A- 0 183 524
- EP-A- 0 319 922
- EP-A- 0 376 111
- EP-A- 0 492 326

## Beschreibung

Die Erfindung betrifft ein Auswertegerät für Teststreifen. Es hat eine Teststreifenhalterung, um den Teststreifen in einer definierten Meßposition gegenüber einer Meßeinheit zu positionieren.

Zur Analyse von Flüssigkeiten, insbesondere von Körperflüssigkeiten wie Blut oder Urin, werden häufig Teststreifenanalysesysteme eingesetzt. Dabei wird zwischen Ein-Parameter-Systemen, mit denen nur eine bestimmte Analyse (beispielsweise Glukose im Blut) durchgeführt werden kann und Mehr-Parameter-Systemen unterschieden, die mit mehreren verschiedenen jeweils auf eine Analyse abgestimmten Testträgertypen arbeiten, die alle mit dem gleichen Gerät ausgewertet werden.

Die Teststreifen bestehen üblicherweise aus einer länglichen Basisschicht und mindestens einem in einem Testfeldbereich darauf angeordneten Testfeld. Das Testfeld enthält ein oder mehrere Reagenzien. Bringt man diese mit der Probe in Kontakt, so läuft eine Reaktion ab, die schließlich zu einem nachweisbaren Signal, insbesondere zu einer Farbänderung in einer Detektionsschicht führt. Durch eine reflexionsphotometrische Messung im Auswertegerät, bei der das diffuse Reflexionsvermögen der Testfeldoberfläche gemessen wird, kann auf die Konzentration des zu bestimmenden Bestandteils der Flüssigkeit geschlossen werden.

Um beim Einführungsvorgang dem Teststreifen eine Zwangsrichtung vorzugeben und zur Querpositionierung in der Meßposition weist die Teststreifenhalterung eine Führung auf. Für die exakte Positionierung der Teststreifen gegenüber dem Reflexionsphotometer sind in den Auswertegeräten üblicherweise Positioniereinrichtungen vorhanden. Die Genauigkeit der Messung und die Einfachheit der Handhabung werden wesentlich durch die Positioniereinrichtung bestimmt. Die Positionierung des Teststreifens in der Teststreifenhalterung bezieht sich auf alle drei Raumrichtungen, nämlich auf die Längs- und Querrichtung des Testfeldes und auf die Richtung vertikal zu der Testfeldoberfläche.

Der vertikale Abstand der Oberfläche des Testfeldes von der Meßoptik ist ein entscheidender Parameter für eine exakte Messung. Da aus Kostengründen in zunehmendem Maße dazu übergegangen wird, die Testfeldflächen immer kleiner zu machen, muß auch die Längs- und Querpositionierung der Teststreifen sehr exakt vorgenommen werden, um einen möglichst großen Teil der Oberfläche der Detektionsschicht als Meßfläche verwenden zu können. Eine falsche räumliche Ausrichtung der Teststreifen führt unmittelbar zu einer Verkleinerung der effektiven Meßfläche und dadurch zu einem Meßfehler.

Aus der EP-A 0 376 111 (US-Patent 5,091,154) ist ein Teststreifenanalysesystem bekannt, bei dem die Teststreifen im Bereich ihres Vorderendes eine Ausnehmung aufweisen. Die Positioniereinrichtung des Auswertegerätes besitzt einen drehbar gelagerten konischen Nocken, der beim Einführen des Teststreifens in das Auswertegerät in dessen Ausnehmung geschwenkt wird. In der Positionierungsendlage stößt der Teststreifen mit seinem Vorderende an einen Anschlag an und der schwenkbare konische Nocken drückt ihn auf eine Auflagefläche nieder. Dabei greift der Nocken in die Ausnehmung derart ein, daß der Teststreifen in allen drei Raumrichtungen kraftbelastet ist, wodurch eine Positionierung erreicht wird.

In einer weiteren, durch die EP-A 0 129 220 (US-Patent 4,780,283) bekannten Vorrichtung wird ein Teststreifen mit jeweils einer Ausnehmung an seinem Handhabungs- und Vorderende verwendet, in die jeweils ein Spannstift eingreift. Beim Positioniervorgang wird zunächst der Spannstift am Vorderende in die entsprechende Ausnehmung bewegt. Danach wird durch die Betätigung einer verschiebbaren Klappe der Teststreifen gebogen, so daß der sich im Bereich des Handhabungsende befindende Spannstift in die zweite Ausnehmung eingreift. An den zweiten Spannstift greift eine Feder derart an, daß der Teststreifen in Längsrichtung unter Zugspannung steht. Durch diese Zugspannung wird der Teststreifen mit seiner Unterseite gegen eine Andruckplatte gedrückt, wodurch das auf der Oberseite des Teststreifens angeordnete Testfeld in der gewünschten Position ist.

Bei den bekannten Teststreifenauswertesystemen wurde für die Teststreifen-Positioniereinrichtungen ein hoher konstruktiver Aufwand mit verschleißanfälligen mechanischen Teilen betrieben. Außerdem ist es für die Funktionsfähigkeit der bekannten Positioniereinrichtung notwendig, die Ausnehmungen an den Teststreifen exakt zu positionieren.

Der vorliegenden Erfindung liegt das technische Problem zugrunde, eine genaue Positionierung der Teststreifen gegenüber einer Meßeinheit in dem Auswertegerät in Richtung vertikal zu der Testfeldoberfläche mit einfacher und sicherer Handhabung zu ermöglichen, wobei dies mit möglichst geringem konstruktiven Aufwand erreicht werden soll.

Dieses Problem wird erfindungsgemäß gelöst durch ein Teststreifenauswertegerät gemäß Anspruch 1 gelöst.

Im Rahmen der Erfindung wurde gefunden, daß eine Biegung des Teststreifens um eine quer zu seiner Längsachse und parallel zu seiner Oberfläche orientierte Biegeachse für die Positionierung genutzt werden kann. Die Biegespannung entsteht dabei durch die Elastizität der Basisschicht des Teststreifens. Als Teststreifen im Sinne der Erfindung sollen daher alle Analyseelemente (Testträger) verstanden werden, die aufgrund ihrer Materialeigenschaften und Dimensionen eine ausreichende Elastizität für eine derartige Positionierung haben.

Bei dem erfindungsgemäßen Teststreifenauswertegerät wird eine Positionierung und Fixierung des Teststreifens in der vordefinierten Meßposition alleine durch die Einführbewegung der Teststreifen in die Teststreifenhalterung ermöglicht. Es muß weder durch den Benutzer noch durch einen Antrieb des Auswertegerätes eine Mechanik zur Fixierung der Teststreifen im Auswertegerät betätigt werden.

Von besonderem Vorteil ist, daß die exakte Positionierung des Testfeldes ohne irgendwelche in der Nähe des Testfeldbereiches von oben auf den Teststreifen drückende Teile erreicht wird. Bei vorbekannten Geräten befindet sich die Teststreifenhalterung vielfach unter einer Klappe mit Haltefedern, die von oben auf den Teststreifen drücken. Das Öffnen und Schließen dieser Klappe ist ein zusätzlicher Handhabungsschritt, der auch Fehlerrisiken birgt, wenn die Klappe versehentlich.nicht vollständig geschlossen wird.

Bei der Erfindung wird eine präzise Positionierung mit extrem geringem Aufwand, sowohl hinsichtlich der Teststreifenhalterung als auch der Teststreifen selbst, erreicht.

Gemäß einer bevorzugten Ausführungsform ist die Teststreifenhalterung so ausgebildet, daß sie in der Meßposition die erste Seite des Teststreifens zwischen der Abstützung und dem Andruckelement nicht berührt, so daß der Teststreifen zwischen der Abstützung und dem Andruckelement frei gespannt ist.

In einer weiteren bevorzugten Ausgestaltung ist das Andruckelement stationär. Damit kommt die Teststreifenhalterung völlig ohne bewegliche Teile aus.

Die Erfindung wird im folgenden anhand von in den Figuren schematisch dargestellten Ausführungsbeispielen näher erläutert; es zeigen:
- Fig. 1: Eine perspektivische Darstellung eines Teststreifenanalysesystems mit einem erfindungsgemäßen Teststreifenauswertegerät;
- Fig. 2: eine perspektivische Darstellung einer Teststreifenhalterung;
- Fig. 3: eine Schnittdarstellung längs der Linie III-III aus Fig. 2 von einer Teststreifenhalterung mit einem Teststreifen in Meßposition;
- Fig. 4: einen Ausschnitt einer zweiten Ausgestaltung der Teststreifenhalterung in einer Schnittdarstellung entsprechend Fig. 3.

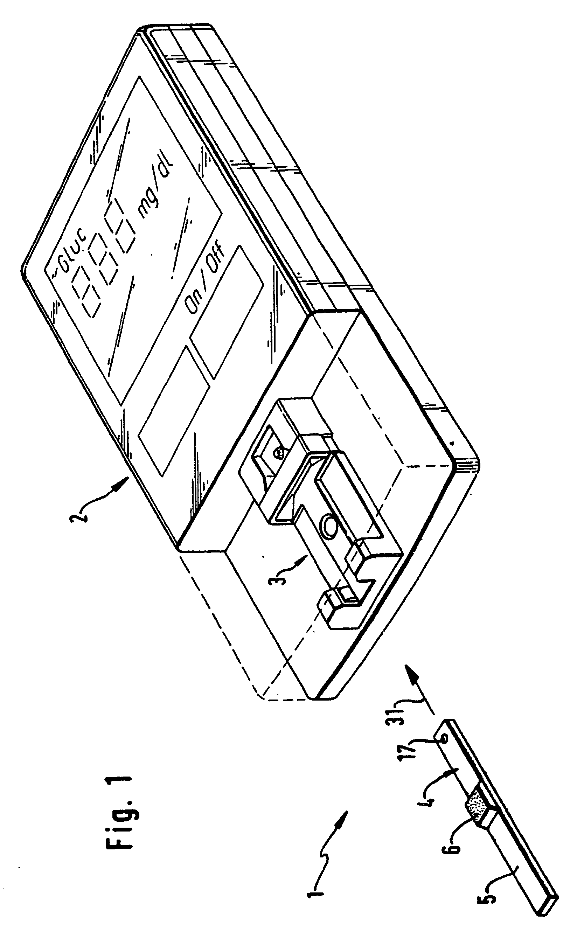

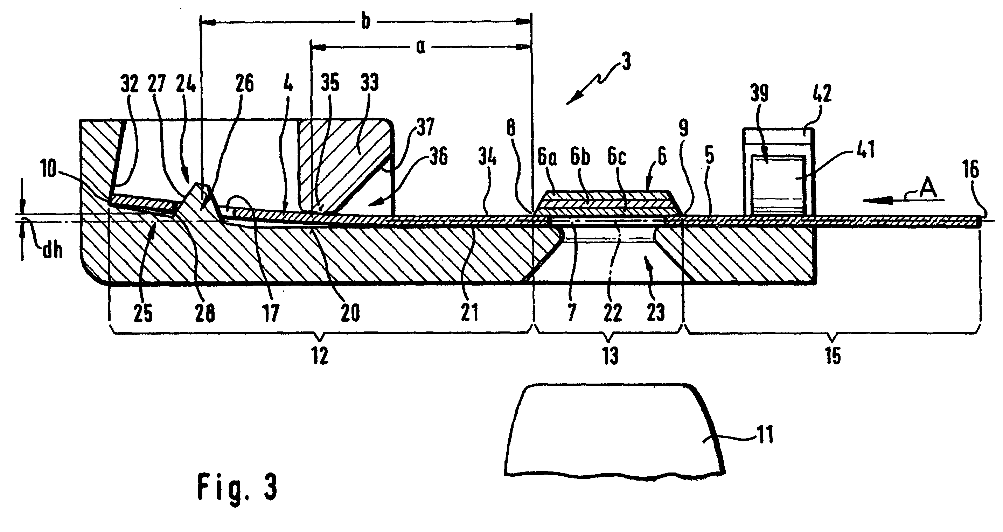

Figur 1 und Figur 3 zeigen ein Teststreifenanalysesystem 1, das ein Auswertegerät 2 mit einer Teststreifenhalterung 3 und Teststreifen 4 umfaßt. Die Teststreifen 4 (von denen nur einer dargestellt ist) weisen eine elastische Basisschicht 5, die üblicherweise aus einem Kunststoffmaterial besteht und ein Testfeld 6 auf. Der dargestellte Teststreifen 4 ist ein sogenannter "nicht abzuwischender (non wipe)" Teststreifen. Bei diesen Teststreifen durchläuft die Körperflüssigkeit, nachdem sie auf der Oberseite des Testfeldes aufgebracht ist, die gesamte Dicke des aus mehreren Schichten 6a-6c (Fig.3) bestehenden Testfeldes 6. Dabei finden chemische Reaktionen zwischen der Körperflüssigkeit und den in dem Testfeld 6 enthaltenen Reagenzien statt. Eine resultierende optisch nachweisbare Veränderung in einer Detektionsschicht 6c kann von der Unterseite 21 des Teststreifens her reflexionsphotometrisch detektiert werden. Wie in Figur 3 zu erkennen ist, hat die Basisschicht 5 des Teststreifens 4 im Bereich des Testfeldes 6 zu diesem Zweck eine Meßöffnung 7.

Der Teststreifen 4 kann in seiner Längsrichtung in drei Abschnitte unterteilt werden. Das Testfeld 6 definiert einen Testfeldbereich 13. Da der Teststreifen 4 im dargestellten Fall nur ein Testfeld 6 aufweist, wird der Testfeldbereich 13 durch die vordere Kante 8 und hintere Kante 9 des Testfeldes 6 begrenzt. Die Erfindung eignet sich jedoch auch für Teststreifen, bei denen in einem größeren Testfeldbereich mehrere Testfelder hintereinander angeordnet sind. Der Testfeldbereich erstreckt sich in diesem Fall in Einführrichtung A von der vorderen Kante des ersten Testfeldes bis zu der hinteren Kante des letzten Testfeldes. Der Abschnitt zwischen dem Vorderende 10 (mit welchem der Teststreifen in die Teststreifenhalterung 3 eingeführt wird) und dem Testfeldbereich 13 wird als Vorderabschnitt 12 bezeichnet. Zwischen dem (dem Vorderende 10 gegenüberliegenden) Handhabungsende 16 des Teststreifens 4 und dem Testfeldbereich 13 erstreckt sich ein Handhabungsabschnitt 15. Der Teststreifen 6 weist im Vorderabschnitt 12 in der Nähe des Vorderendes 10 eine kreisrunde Ausnehmung 17 auf, die in Querrichtung des Teststreifens 6 mittig angeordnet ist.

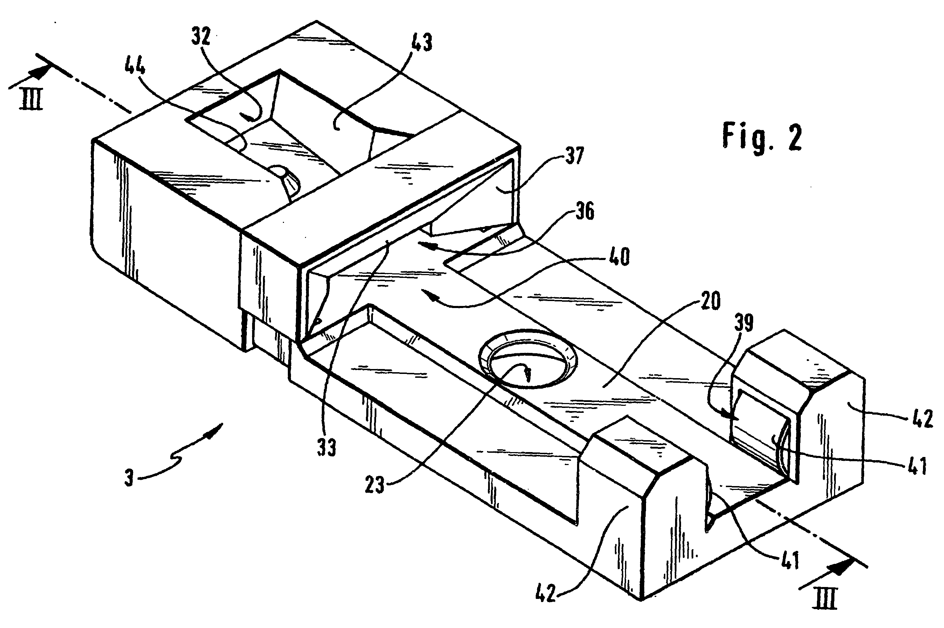

Wie in den Figuren 2 und 3 zu erkennen ist, besitzt die Teststreifenhalterung 3 eine Teststreifenauflage 20 auf der der Teststreifen 4 mit einer ersten Seite (Unterseite) 21 aufliegt. In der in Figur 3 dargestellten Meßposition befindet sich der Teststreifen 4 derartig auf der Teststreifenauflage 20, daß die Mittelebene 22 des Testfeldbereiches 13 einen definierten Abstand von einer Meßeinheit 11 aufweist, welche sich unterhalb der Meßöffnung 23 der Teststreifenauflage 20 befindet. Als Mittelebene 22 des Testfeldbereiches 13 wird dabei, wie in Fig. 3 zu erkennen ist, die im Testfeldbereich 13 durch die Mitte der Basisschicht 5 des Teststreifens 4 verlaufende geometrische Ebene bezeichnet.

Ein Teil der Teststreifenauflage 20 ist in dem Bereich, in dem sich in der Meßposition der Vorderabschnitt 12 befindet, als Abstützung 24 ausgebildet, welche in der Höhe gegenüber der Mittelebene 22 des Testfeldbereiches 6 versetzt ist. Mit anderen Worten ist zwischen der Stelle, an der der Teststreifen auf der Abstützung 24 aufliegt, und der Mittelebene 22 ein definierter vertikaler Abstand vorhanden. In der dargestellten Ausführungsform dient ein stationärer kegelförmiger Haltedorn 26 zugleich als Abstützung 24 und als Fixierungselement 25 zur Fixierung der Position des Teststreifens 4 in seiner Längsrichtung, wobei die Einführbewegung des Teststreifens 4 durch einen Anschlag 32 begrenzt wird.

Für diese Funktion des Haltedorns 26 ist wichtig, daß er eine zu der Oberfläche des Teststreifens (im Bereich der Ausnehmung 17) in Richtung auf den Anschlag 32 geneigte (in einem spitzen Winkel zu der Teststreifenoberfläche verlaufende) Mantelfläche 27 aufweist und die Teststreifenauflage 20 in der Umgebung des Haltedorns 26 so gestaltet ist, daß der Teststreifen 4 in seinem dem Vorderende 10 zugewandten vordersten Abschnitt nur zwei definierte Berührungsstellen hat, nämlich daß er mit seinem Vorderende 10 an dem Anschlag 32 anstößt und mit einem dem Vorderende 10 zugewandten Abschnitt 28 des Randes der Ausnehmung 17 auf der geneigten Mantelfläche 27 des Haltedorns 26 ruht. Der Abstand zwischen der Mitte des Haltedorns 26 und dem Anschlag 32 ist etwas kleiner als der Abstand zwischen der Mitte der Ausnehmung 17 und dem Vorderende 10 des Teststreifens 4.

Infolge dieser Konstruktionsweise ist die Bewegungsmöglichkeit des Teststreifens 4 (nach unten) in Richtung auf die Teststreifenauflage 20 (im Bereich des Haltedorns 26) durch die von der Mantelfläche 27 gebildete Abstützung 24 begrenzt. Der Abstand der Abstützung 24 von der Mittelebene 22 des Testfeldbereiches 13 ist in der Figur mit dh bezeichnet. Die Abstützung 24 ist in diesem Falle also um dh gegenüber der Mittelebene 22 versetzt.

Die Teststreifenhalterung 3 weist ein stationäres Andruckelement 33 auf, das die Teststreifenauflage 20 brückenartig überspannt. Die Höhe des zwischen dem stationären Andruckelement 33 und der Teststreifenauflage 20 gebildeten Spalts 36, durch den der Teststreifen 4 beim Einführen in die Teststreifenhalterung 3 hindurchgeführt wird, ist mindestens 1,5 mal so groß wie die Dicke der Basisschicht 5. Das Andruckelement 33 drückt dabei etwa in der Mitte des Vorderabschnittes 12 gegen die zweite Seite (Oberseite) 34 des Teststreifens 4. Die Fläche 35, mit der das Andruckelement 33 den Teststreifen 4 berührt, ist näher bei der Mittelebene 22 als die Abstützung 24, so daß der Teststreifen 4 in der Meßposition unter Biegespannung steht. Durch die Elastizität der Basisschicht 5 wird der Teststreifen im Testfeldbereich 13 gegen die Teststreifenauflage 20 gedrückt und somit der definierte Abstand des Testfeldes 6 von der Meßeinheit 11 sichergestellt. Um die Einführung des Teststreifens 4 in den Spalt 36 zu vereinfachen, ist die stirnseitige Fläche 37 des Andruckelementes 33 trichterförmig ausgebildet.

Durch die Elastizität der Basisschicht 5 und die beschriebenen geometrischen Verhältnisse wird auch das Vorderende 10 des Teststreifens 4 nach unten gegen den Haltedorn 26 gedrückt. Hierdurch wird zugleich eine Positionierung des Teststreifens 4 in Längsrichtung bewirkt, weil die Ausnehmung 17 auf der geneigten Mantelfläche 27 in Richtung auf den Anschlag 32 gleitet und somit (durch die eigene Elastizität der Basisschicht 5) eine Kraftkomponente in Längsrichtung des Teststreifens 4 auf den Anschlag 32 hin wirkt.

Bevorzugterweise beträgt der Abstand a des stationären Andruckelementes 33 von der dem Vorderende 10 des Teststreifens 4 zugewandten Begrenzung des Testfeldbereiches 13 zwischen dem 0,35 fachen und dem 0,65 fachen des Abstandes b zwischen der dem Vorderende 10 zugewandten Begrenzung des Testfeldbereiches 13 und der Abstützung 24.

Wie in Figur 3 zu erkennen ist, ist die Länge der Teststreifenauflage 20 kürzer als die Länge des Teststreifens 4, so daß nur ein dem Testfeldbereich 13 zugewandter Teil des Handhabungsabschnittes 15 auf der Teststreifenauflage 20 aufliegt. Der andere Teil des Handhabungsabschnittes 15 ragt frei aus dem Auswertegerät 2 heraus, was die Handhabung des Teststreifens 4 erleichtert.

Wie in Figur 2 dargestellt, weist die Teststreifenhalterung 3 zum Einführen der Teststreifen 4 und zur exakten Querpositionierung eine Führung 40 auf. In Abhängigkeit von der Steifigkeit der Basisschicht der Teststreifen kann diese im Bereich des Handhabungsabschnitts 15 unterschiedlich ausgestaltet sein. Ist die Basisschicht relativ steif, so ist es ausreichend, wenn die Führung in diesem Bereich Querbewegungen der Teststreifen verhindert. Dies kann beispielsweise durch zwei Führungselemente mit zur Einführrichtung parallelen und sich senkrecht von der Teststreifenauflage erhebenden Führungsflächen erreicht werden, deren Abstand voneinander etwas größer ist als die Breite der Teststreifen.

Bei dünnen und deswegen flexiblen Teststreifen kann es zur sicheren Auflage der Teststreifen auf der Teststreifenauflage 20 vorteilhaft sein, wenn die Führung 40 den Teststreifen zusätzlich zur seitlichen Führung auf die Teststreifenauflage 20 drückt. Bei dem in den Figuren 1 bis 3 dargestellten Ausführungsbeispiel ist im Bereich des Handhabungsabschnittes 15 seitlich am Ende der Teststreifenauflage 20 je ein elastisches Federelement 39 in Form von elastischen Biegefedern 41 angeordnet. Diese sind in entsprechenden Aufnahmen 42 befestigt. Die Biegefedern 41 ragen jeweils mit einem kreissegmentförmigen Profil aus den Aufnahmen 42 heraus, wobei sie oberhalb der Teststreifenauflage 20 den geringsten Abstand voneinander haben. In Höhe eines auf der Teststreifenauflage 20 liegenden Teststreifens ist ihr Abstand etwas geringer als dessen Breite. Dadurch kann ein Teststreifen 4 zwischen den Biegefedern 41 elastisch festgeklemmt und fixiert werden.

Die Führung 40 im Bereich des Vorderabschnittes 12 wird durch die Teststreifenauflage 20 begrenzende Wandflächen 43,44 gebildet, die sich senkrecht von der Teststreifenauflage 20 erheben. Vom Andruckelement 33 ausgehend bilden die Wandflächen 43,44 zuerst eine konisch zulaufende Seitenführung, um dann mit konstantem Abstand voneinander, der geringfügig größer als die Breite eines Teststreifens 4 ist, bis zum Anschlag 32 zu verlaufen.

Soll ein Teststreifen 4 in das Auswertegerät 2 eingeführt werden, so wird er mit seinem Vorderende 10 schräg von oben in den Spalt 36 der Teststreifenhalterung 3 gesteckt. Bei der weiteren Einführbewegung wird das Vorderende 10 durch den gegenüber der Teststreifenauflage 20 erhabenen Haltedorn 26 angehoben. Sobald der Haltedorn 26 in die Ausnehmung 17 eingreift, entsteht durch die nach unten wirkende elastische Spannung des Teststreifens 4 und die schräge Neigung der Mantelfläche 27 des Haltedorns 26 die beschriebene Kraftkomponente auf den Anschlag 32 zu. Der Teststreifen 4 rutscht in Richtung auf den Anschlag 32 bis er die in Figur 3 dargestellte definierte Meßposition erreicht hat.

Das Handhabungsende 16 wird manuell gegen den Widerstand der Biegefedern 41 nach unten gegen die Teststreifenauflage 20 gedrückt und dort durch die Biegefedern 41 fixiert.

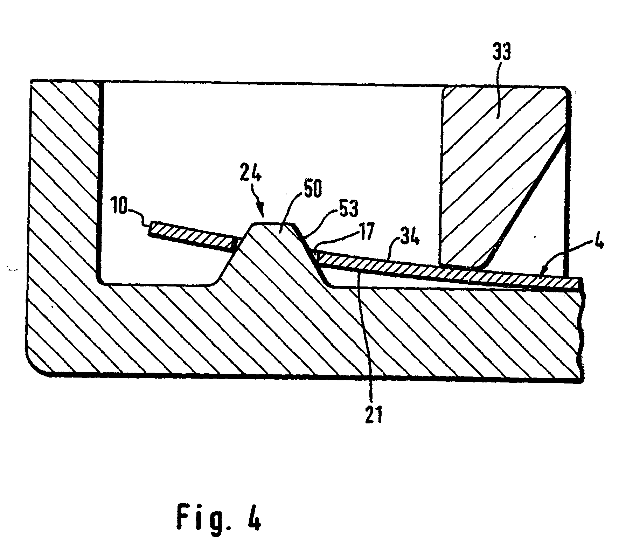

In Figur 4 ist ausschnittsweise eine alternative zweite Ausführungsform der Erfindung dargestellt. Der Unterschied zur zuvor beschriebenen Ausführungsform besteht im wesentlichen darin, daß dieses Teststreifenauswertegerät ohne einen Anschlag im Bereich des Vorderendes auskommt. Alle anderen Merkmale des zuvor beschriebenen Ausführungsbeispieles sind auch bei diesem verwirklicht. Der Haltedorn 50 und die Ausnehmung 17 des Teststreifens 4 haben kreisrunde Querschnitte, wodurch der Teststreifen 4 in seiner Meßposition zentrisch und mit dem Rand der Ausnehmung 17 im wesentlichen vollständig auf der Mantelfläche 53 des Haltedorns 50 aufliegt und dadurch fixiert ist.

Bei den in Figur 3 und Figur 4 dargestellten Ausführungsformen wird die Funktion der Abstützung und des Fixierungselementes gemeinsam durch den kegelförmig ausgebildeten Haltedorn 26,50 übernommen. Beide Funktionen können jedoch auch von getrennten Elementen erfüllt werden. Beispielsweise wäre es möglich, die Teststreifenauflage 20 in Figur 3 in Richtung auf den Anschlag 32 gleichmäßig nach oben gekrümmt zu gestalten, um dadurch die gewünschte gegenüber der Mittelebene 22 des Testfeldbereiches 13 versetzte Abstützung zu bewirken. Die Fixierung in Längsrichtung könnte dabei an anderer Stelle, beispielsweise in dem auf der Auflage 20 aufliegenden Teilbereich des Handhabungsabschnittes 15 mit Hilfe einer dort in dem Teststreifen vorgesehenen Ausnehmung und einer entsprechenden Fixierungseinrichtung bewirkt werden.

## Patentansprüche

1. Teststreifenauswertegerät zur Auswertung von Teststreifen (4), die eine Basisschicht (5) aufweisen, an der in einem Testfeldbereich (13) wenigstens ein Testfeld (6) fixiert ist,
mit einer Teststreifenhalterung (3), um einen Teststreifen (4) in einer definierten Meßposition gegenüber einer Meßeinheit (11) zu positionieren,
wobei
die Teststreifenhalterung (3) eine Führung (40) aufweist, durch die ein Teststreifen (4) beim Einführen in die Teststreifenhalterung in seiner Querrichtung geführt wird, und
eine erste Seite eines in der Meßposition befindlichen Teststreifens (4) in seinem Testfeldbereich (13) auf einer Teststreifenauflage (20) mit einer Meßöffnung (23) derartig aufliegt, daß sein Testfeldbereich (13) einen definierten Abstand von einer unterhalb der Meßöffnung (23) befindlichen Meßeinheit (11) hat,
**dadurch gekennzeichnet, daß**
die Teststreifenhalterung (3) eine gegenüber der durch die Mitte der Basisschicht (5) des Teststreifens (4) in dem Testfeldbereich(13) verlaufenden geometrischen Ebene (22) vertikal versetzte Abstützung (24) und ein Andruckelement (33) aufweist, wobei in der Meßposition das Andruckelement (33) derartig zwischen der Abstützung (24) und dem Testfeldbereich (13) gegen die zweite Seite (34) des Teststreifens (4) drückt, daß der Teststreifen (4) in der Meßposition unter Biegespannung in Richtung auf die Meßöffnung steht, wodurch er in dem Testfeldbereich (13) gegen die Teststreifenauflage (20) mit der Meßöffnung (23) gedrückt und somit der definierte Abstand des mindestens einen Testfeldes (6) von der Meßeinheit (11) sichergestellt wird.

2. Teststreifenauswertegerät nach Anspruch 1 **dadurch gekennzeichnet, daß** die Teststreifenhalterung (3) so ausgebildet ist, daß sie in der Meßposition die erste Seite (21) des Teststreifens (4) zwischen der Abstützung (24) und dem Andruckelement (33) nicht berührt, so daß der Teststreifen (4) zwischen der Abstützung (24) und dem Andruckelement (33) frei gespannt ist.

3. Teststreifenauswertegerät nach einem der vorhergehenden Ansprüche, **dadurch gekennzeichnet, daß** das Andruckelement (33) stationär ist, wobei darunter ein Spalt (36) gebildet wird, durch den der Teststreifen (4) beim Einführen in die Teststreifenhalterung (3) hindurchgeführt wird.

4. Teststreifenauswertegerät nach Anspruch 3, **dadurch gekennzeichnet, daß** der Spalt zwischen dem stationären Andruckelement (33) und der Teststreifenauflage (20), durch den der Teststreifen beim Einführen in die Teststreifenhalterung (3) hindurchgeführt wird, mindestens 1,5 mal so groß wie die Dicke der Basisschicht (5) eines in der Meßposition befindlichen Teststreifens (4) ist.

5. Teststreifenauswertegerät nach einem der vorhergehenden Ansprüche, **dadurch gekennzeichnet, daß** die Teststreifenhalterung (3) beidseitig der Führung (40) elastische Federelemente (39) aufweist, die den Test streifen (4) in einem Handhabungsabschnitt (15) zwischen dem Testfeldbereich (13) und einem Handhabungsende (16) des Teststreifens (4) durch Druck gegen seine Kanten gegen die Teststreifenauflage (20) drücken.

## Claims

1. Test strip evaluation device for evaluating test strips (4) which comprise a base layer (5) to which at least one test field (6) is fixed in a test field area (13),
with a test strip holder (3) for positioning a test strip (4) in a defined measuring position relative to a measuring unit (11),
wherein
the test strip holder (3) comprises a guide (40) for laterally guiding a test strip (4) when it is inserted into the test strip holder and
a first side of a test strip (4) which is in the measuring position rests in its test field area (13) on a test strip seat (20) having a measuring aperture so that its test field area (13) is at a defined distance from a measuring unit (11) below the measuring aperture (23),
**characterized in that**
the test strip holder (3) comprises a support (24) vertically offset relative to the geometric plane (22) extending in the test field area (13) through the middle of the base layer (5) of the test strip (4), and a pressure element (33) which in the measuring position presses against the second side (34) of the test strip (4) at a position which is located between the support (24) and the test field area (13), in such a way that the test strip (4) is in the measuring position subjected to bending stress so that the test strip (4) in the test field area (13) is pressed against the test strip seat (20) with the measuring aperture (23) and thus the defined distance of the at least one test field (6) from the measuring unit (11) is ensured.

2. Test strip evaluation device according to claim 1, **characterized in that** the test strip holder (3) is so designed that in the measuring position it does not contact the first side (21) of the test strip (4) between the support (24) and the pressure element (33), so that the test strip (4) is tensioned freely between the support (24) and the pressure element (33).

3. Test strip evaluation device according to any one of the preceding claims, **characterized in that** the pressure element (33) is stationary and a gap (36) is formed under the pressure element (33) through which gap the test strip (4) is passed on insertion into the test strip holder (3).

4. Test strip evaluation device according to claim 3, **characterized in that** the gap between the stationary pressure element (33) and the test strip seat (20), through which gap the test strip is passed on insertion into the test strip holder (3) is at least 1.5 times as thick as the base layer (5) of a test strip (4) in the measuring position.

5. Test strip evaluation device according to any one of the preceding claims, **characterized in that** the test strip holder (3) comprises elastic spring elements (39) located on both sides of the guide (40) and pressing on the edges of the test strip (4), thereby pressing the test strip (4) in a handling section (15) between its test field area (13) and its handling end (16) against the test strip seat (20).

## Revendications

1. Système d'analyse de bande de test pour l'analyse de bandes de test (4) qui présentent une couche de base (5) sur laquelle est fixé au moins un champ de test (6) dans une zone de champ de test (13),
avec un élément porteur de bande de test (3) afin de positionner une bande de test (4) dans une position de mesure définie par rapport à une unité de mesure (11),
l'élément porteur de bande de test (3) présentant un guide (40) à travers lequel la bande de test (4) est guidée dans le sens transversal lors de son introduction dans l'élément porteur de bande de test et selon lequel
une première face d'une bande de test (4) placée en position de mesure dans sa zone de champ de test (13) repose sur une surface de pose de bande de test (20) munie d'une ouverture de mesure (23) de sorte que sa zone de champ de test (13) est à une distance définie de l'unité de mesure (11) qui se trouve au-dessous de l'ouverture de mesure (23)
**caractérisé en ce que**
l'élément porteur de la bande de test (3) présente un élément de compression (33) et un support (24) décalé verticalement par rapport au plan géométrique (22) s'étendant dans la zone de champ de test (13) à travers le milieu de la couche de base (5) de la bande de test (4), moyennant quoi l'élément de compression (33) en position de mesure appuie sur la deuxième face (34) de la bande de test (4), entre le support (24) et la zone de champ de test (13), de telle façon que la bande de test (4) en position de mesure est tournée vers l'ouverture de mesure sous contrainte de flexion, grâce à laquelle elle est comprimée dans sa zone de champ de test (13) contre la surface de pose de bande de test (20) munie d'une ouverture de mesure (23), et la distance définie d'au moins un champ de test (6) par rapport à l'unité de mesure (11) est ainsi assurée.

2. Système d'analyse de bande de test selon la revendication 1, **caractérisé en ce que** l'élément porteur de bande de test (3) est conçu de telle façon qu'en position de mesure, il ne touche pas la première face (21) de la bande de test (4) entre le support (24) et l'élément de compression (33), si bien que la bande de test (4) est tendue librement entre le support (24) et l'élément de compression (33).

3. Système d'analyse de bande de test selon l'une quelconque des revendications précédentes, **caractérisé en ce que** l'élément de compression (33) est fixe, au-dessous duquel une fente (36) est formée à travers laquelle la bande de test (4) est insérée lors de son introduction dans l'élément porteur de bande de test (3).

4. Système d'analyse de bande de test selon la revendication 3, **caractérisé en ce que** la fente entre l'élément de compression fixe (33) et la surface de pose de bande de test (20), fente à travers laquelle la bande de test est insérée lors de son introduction dans l'élément porteur de bande de test (3), est au moins 1,5 fois plus grande que l'épaisseur de la couche de base (5) d'une bande de test (4) se trouvant en position de mesure.

5. Système d'analyse de bande de test selon l'une quelconque des revendications précédentes, **caractérisé en ce que** l'élément porteur de bande de test (3) présente des deux côtés du guide (40) des éléments élastiques à ressort (39) qui compriment contre la surface de pose de bande de test (20) la bande de test (4) par pression contre les bords de celle-ci dans un segment préhensible (15) entre la zone de champ de test (13) et la section préhensible (16) de la bande de test (4).
